# EUROPEAN PATENT SPECIFICATION

(11) **EP 3 570 979 B1**
(45) Date of publication and mention of the grant of the patent: **14.07.2021**
(21) Application number: 18703905.2
(22) Date of filing: 18.01.2018
(51) Int. Cl.: G01N 15/00, B01L 3/00, B01L 7/00, G01N 35/08, B01F 13/00, C12Q 1/6844, G01N 15/14, G01N 15/10

(54) **MICROFLUIDIC SYSTEM AND METHOD WITH TIGHTLY CONTROLLED INCUBATION TIME AND CONDITIONS**
MIKROFLUIDISCHES SYSTEM UND VERFAHREN MIT GENAU GESTEUERTEN INKUBATIONSZEITEN UND ZUSTÄNDEN
SYSTÈME MICROFLUIDIQUE ET PROCÉDÉ AVEC DURÉE ET ÉTATS D'INCUBATION ÉTROITEMENT RÉGULÉS

(30) Priority: 18.01.2017 EP 17151966
(43) Date of publication of application: 27.11.2019
(73) Proprietor: Biomillenia SAS, 75005 Paris (FR)
(72) Inventor: DU, Guansheng, 93230 Romainville (FR); LÖFFERT, Dirk, 42781 Haan (DE); SHIUE, Eric, Burlington MA 01803-4437 (US)
(74) Representative: CH Kilger Anwaltspartnerschaft mbB
(86) International application number: PCT/EP2018/051222
(87) International publication number: WO 2018/134311

(56) References cited:
- EP-A1- 3 000 528
- WO-A1-2016/020414
- US-A1- 2007 195 127
- US-A1- 2008 014 589
- US-A1- 2012 121 480

## Description

### FIELD OF THE INVENTION

The present invention is in the field of microfluidic systems and methods that use such systems. More specifically, the microfluidic system of the invention is a system with an external delay line that allows the precise control of the incubation time and conditions of droplets that flow through the delay line. The methods are high throughput screening or *in vitro* evolution methods.

### BACKGROUND

Microfluidic devices are powerful tools that allow to miniaturise and to perform a large number of assays in parallel. As a consequence, microfluidic devices are ideal tools to vastly increase the throughput of many types of laboratory assays, such as screenings analyses or *in vitro* evolution.

Microfluidic devices are essentially networks of small channels used for the precise manipulation of small amounts of fluids. Miniaturised reaction vessels, which are in facts droplets of fluid, flow through the channels of the microfluidic system. Along their flow path, the droplets can be manipulated. A reagent can for example be added to at least a subset of the droplets by various methods known in the art, such as nanoinjection. Such a reagent can for example be a substrate for an enzymatic reaction which becomes fluorescent if an enzyme with desired properties is present in the droplet. In such a setup, the droplets are incubated and the fluorescence of the individual droplets is measured. The droplets with the highest level of fluorescence, can then be selected. This allows for example screening a large number of different enzymes with random mutations. As an example, EP3000528A1 discloses a microfluidic device and a method allowing the generating and screening of combinatorial samples.

The assays used in such methods often require a precise incubation period after mixing of all of the reagents. For example, to measure the kinetics of an enzymatic reaction inside the droplets, it is required to incubate the fluorescent enzyme substrate and enzyme for an optimised period of time. In such an experiment, it is important that each of the droplets be incubated for exactly the same amount of time. This is only possible, in the context of a microfluidic device, if it takes the different droplets the same amount of time to flow through the microfluidic system.

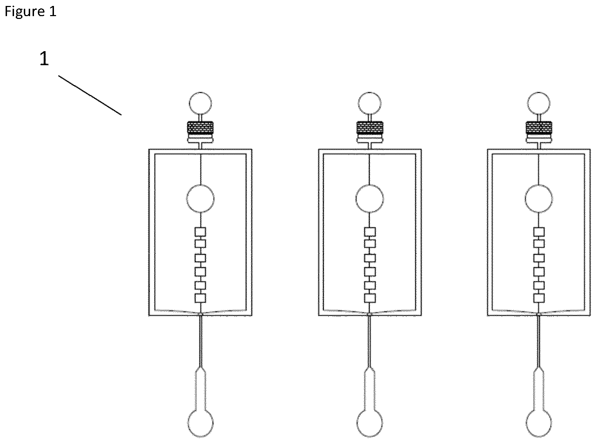

Several solutions have been put forward in the art to solve this problem. US 2012 0121480 A1 for example proposes a system with a delay line that has deep channels in which many droplets flow in parallel (see for example figure 1). The deep channels allow prolonged incubation times. The drawback of this solution is a relatively large dispersion rate of the droplets. Indeed, due to the phenomenon of laminal flow in the tubing, the droplets at the centre of tubing move faster than the droplets at the side of the tubing. Therefore, the incubation time of the different droplets in the tubing cannot be well controlled. US 2012 0121480 A1 attempts to solve this problem by adding mixing modules which reduce the dispersion rate. However, dispersion cannot be entirely eliminated with such an approach.

US 2008 0014589 A1 also proposes using delay lines to provide an extended incubation time. This document also discusses the problem of dispersion of the individual droplets in the delay line and attempts to solve the problem by using towers, which are structures that are vertical with respect to the ambient gravitational field (see for example paragraphs [303] to [305]). These towers however also fail to eliminate dispersion entirely. As a result, the incubation time of each droplet is not exactly the same. This deviation in incubation times of different droplets is a problem. It is indeed important for many types of assays to conduct the assays under identical conditions across the different droplets. This allows to compare data between individual droplets and, thus, to select droplets with the desired assay outcome.

There is therefore a need in the art for a microfluidic system that allows the precise control of the incubation time and conditions of each of the droplets that flow through it.

### BRIEF DESCRIPTION OF THE INVENTION

The present invention aims to solve this problem by providing a microfluidic system in which the droplets (5) flow through an off-chip delay line (4) in a single file. This ensures that all of the droplets (5) are incubated for precisely the same amount of time and under the same conditions.

The invention therefore relates to a microfluidic system according to claim 1.

In a further aspect, the invention relates to a method for high throughput screening or *in vitro* evolution according to claim 10.

### DETAILED DESCRIPTION OF THE INVENTION

There is a need in the art for a microfluidic system that allows prolonged but tightly controlled incubation times and conditions of assay droplets (5). The inventors have found that this problem can surprisingly be solved by a microfluidic system with an off-chip delay line (4) through which the droplets flow in a linear sequential order (i.e. in a single file). In one embodiment the present invention is therefore a microfluidic system comprising:
a) a chip comprising a droplet-generating device (1) and/or a droplet nanoinjection device (2) and/or a droplet reinjection device and/or a droplet fusion device,
b) an off-chip delay line (4),
c) a droplet-analysis device (7), and
d) optionally a droplet sorting device,
wherein the off-chip delay line (4) is fluidically connected at one end to the outlet (3) of the device a), and at the other end to the inlet (8) of the device c), and
wherein the device a) is configured to generate or provide either:
1) droplets (5) with an unconstrained diameter that is equal or larger than the inner diameter of the off-chip delay line (4); or
2) droplets (5) with an unconstrained diameter that is smaller than the inner diameter of the off-chip delay line (4), in which case the device a) is configured in addition to generate and/or insert, between each droplet (5) or group of about 2 to about 100 droplets (5), a separating-droplet (6) with an unconstrained diameter that is equal or larger than the inner diameter of the off-chip delay line (4), wherein the inner diameter of the off-chip delay line (4) is constant over its entire length, and wherein the inner diameter of the off-chip delay line (4) is between 0.01 mm to 10 mm, preferably between 0.05 mm to 5 mm.

The combination of features of the microfluidic system of the invention ensures that the droplets (5) flow through the delay line (4) in a single file. As a result, the droplets (5) flow through the delay line (4) for precisely the same amount of time and under the same conditions. The inventors have indeed surprisingly found that the problem of dispersion in the delay line (4) can be solved by using a delay line (4) with an inner diameter that is smaller than the unconstrained diameter of the droplets (5). This ensures that the droplets (5) flow through the delay line (4) in a linear sequential order. The inventors have also surprisingly found that the dispersion problem for droplets with an unconstrained diameter smaller than the inner diameter of the delay line can be solved by introducing a separating-droplet (6) between each small droplet or between groups of small droplets. This ensures that the small droplets also flow through the delay line in a linear sequential order. The incubation time of the different types of droplets in the delay line is therefore the same for all the droplets. The solution provided by the system of the invention has clear advantages. It allows to precisely define that the assay time in each droplet is the same from the start of an assay to the detection of the result of the assay. This is particularly critical for assays in which the kinetics of a reaction are important.

The system of the invention allows a more accurate control of the incubation time than the systems of the prior art because dispersion of the droplets is eliminated. The incubation time on microfluidic chips is determined by the size of the droplets and the length of the delay line (sometimes called incubation line). In the prior art, such delay lines have mostly been incubation lines that were present on the chip itself. However, this type of delay line is not well suited for nanolitre-sized droplets (typical droplet volume of nanoliter droplets is from 0.5 nL to 5000 nL) due to the larger volume of nanoliter droplets. For example, to reach the analysis rate of 10 droplets/s using 20 nL droplets, there is only sufficient capacity for delay lines on a typical chip to allow incubation of assay reagents for up to 5 minutes. However, if an incubation time of 15 minutes is required, the delay line would need to be 6 meters long. Such a long delay line cannot be integrated onto a typical chip. This is a problem, as nanoliter droplets are required for many assays that involve cell types which are rather large in size (e.g. algae) or generate filaments such as filamentous fungi or actinomycetes. The problem of a short incubation time also exists for picoliter-sized droplets (the typical volume of picoliter droplets is from 5 pL to 500 pL). Incubation of such droplets in delay lines that are comprised on a chip can be carried out for between 1 minute and 1 hour. The incubation times inside traditional microfluidic systems are therefore limited. This problem is solved by the microfluidic system of the invention by using an off-chip delay line (4) which can be as large as is necessary to provide the required incubation time. A further advantage of the invention is therefore that since the delay-line is an off-chip delay line (4), the length of the delay-line can easily and cheaply be modulated in order to provide longer or shorter incubation times. Another advantage of the microfluidic system of the invention is that the incubation conditions such as the temperature of the off-chip delay line (4) can be specifically controlled, independently of the conditions of the droplet generating device (1) and the droplet analysis device (7).

Droplets (5) with a diameter that is equal or larger than the inner diameter of the off-chip delay line (4) should be understood in the context of this document as meaning that the droplets (5) have an unconstrained diameter that is equal or larger than the inner diameter of the delay line (4). Preferably, the diameter of the droplets (5) is larger than the inner diameter of the delay line (4) by at least 2 %, preferably by at least 5 %, more preferably by at least 10 %, even more preferably by at least 20 %, yet more preferably by at least 30 %, yet more preferably by at least 40 % and most preferably by at least 50 %. As way of example, if the inner diameter of the delay line (4) is 0.2 mm, the device a) is most preferably configured to generate or provide droplets (5) with a diameter of at least 0.3 mm. This definition of the size of the droplet (5) diameter is also applicable to the size of the diameter of the separating-droplets (6).

Droplets (5) with a diameter that is smaller than the inner diameter of the off-chip delay line (4) is to be understood as droplets with a diameter that is smaller relative to the inner diameter of the delay line (4) by at least 2%, preferably by at least 5 %, more preferably by at least 10 %, even more preferably by at least 20 %, yet more preferably by at least 30 %, yet more preferably by at least 40 % and most preferably by at least 50 %. It can also be understood as meaning that the diameter of the droplets (5) is, relative to the inner diameter of the off-chip delay line (4), at most 98%, preferably at most 95 %, more preferably at most 90 %, even more preferably at most 80 %, yet more preferably most 70 %, yet more preferably at most 60 % and most preferably at most 50 %. It should however also be clear that the diameter of the droplets (5) can be much smaller than the inner diameter of the off-chip delay line (4). The diameter of the droplets (5) may indeed represent only 20 %, preferably 10 %, more preferably 5 % and most preferably 1 % or lower of the inner diameter of the off-chip delay line (4).

Preferably, when the device a) is configured to generate or provide droplets (5) with a diameter that is smaller than the inner diameter of the off-chip delay line (4), it is also configured in addition to generate and/or insert between each of these droplets (5), a separating-droplet (6) with a diameter that is equal or larger than the inner diameter of the off-chip delay line (4).

In an alternative embodiment, the device a) of the microfluidic system of the invention is configured to generate and/or insert a separating-droplet (6) between each group of about 2 to about 100 droplets (5) with a diameter that is smaller than the inner diameter of the off-chip delay line (4). Preferably, in this case, it is configured to generate and/or insert such a separating-droplet (6) between each group of 2 to 100 droplets (5) with a diameter that is smaller than the inner diameter of the off-chip delay line (4).

"Separating-droplets" are sometimes referred to as "plugs" in this document. The two phrases are meant to have the same meaning and are therefore interchangeable. The separating-droplets (6) can be made of any phase that is immiscible with the phase of the droplets (5). If the droplets (5) are made of an aqueous phase for example, the separating-droplets (6) can for example be essentially made of an oil phase or a gas phase. An oil phase can for example essentially be a mineral oil, a fluorocarbon oil, a silicon oil, a hydrocarbon oil, a vegetable oil or any combination thereof. Preferably, the separating-droplets (6) are essentially made of a mineral oil.

In one embodiment, the separating-droplets (6) have a volume of 5 nL to 10000 nL, more preferably of 10 nL to 1000 nL, yet more preferably of 15 nL to 500 nL and most preferably of 20 nL. However, as will be clear to the person skilled in the art, the optimal volume of the separating-droplets (6) depends on the inner diameter of the delay line (4). The device must indeed be configured to generate or introduce separating-droplets (6) with a volume for which the unconstrained diameter of the separating-droplets (6) is at least as large as the inner diameter of the delay line (4).

According to the invention, the inner diameter of the off-chip delay line (4) is constant over essentially its entire length. More preferably, the inner diameter of the off-chip delay line (4) is constant over its entire length.

In one embodiment, the length of the off-chip delay line (4) of the microfluidic device is between 0.1 m to 100 m, preferably between 0.5 m to 80 m, more preferably between 1 m to 60 m, even more preferably between 2 m to 40 m, yet more preferably between 5 m and 50 m, yet more preferably between 10 m to 40 m, yet more preferably between 20 m to 30 m, and most preferably the length of the off-chip delay line (4) of the microfluidic device is 25 m. According to the invention, the inner diameter of the delay line (4) is between 0.01 mm to 10 mm, preferably between 0.05 mm to 5 mm, more preferably between 0.1 and 2 mm, even more preferably between 0.2 and 1 mm, yet more preferably between 0.3 and 0.8 mm, and most preferably, the inner diameter of the delay line (4) is 0.5 mm.

In one embodiment of the invention, the off-chip delay line (4) is made of a material selected from the group comprising glass, PTFE (polytetrafluoroethylene), PEEK (polyetheretherketone), FEP (fluorinated ethylene-propylene), ETFE (ethylene tetrafluoroethylene), PP (polypropylene) and any combination thereof. More preferably, the delay line (4) is made of PTFE.

The delay line (4) does not need to be of any particular shape. In a preferred embodiment however, the delay line (4) is coiled. In such an embodiment, the delay line (4) can for example be coiled around a central cylinder. This allows easy handling of the delay line (4). The delay line (4) can in one embodiment also be coiled around a means to control an external condition such as a means that allows to control the temperature of the delay line (4).

In one embodiment of the invention, the microfluidic device is configured to provide a flow rate through the off-chip delay line (4) of 0.001 mL/h to 100 mL/h, preferably of 0.05 mL/h to 50 mL/h, more preferably of 0.1 to 25 mL/h, even more preferably of 0.5 to 20 mL/h, yet more preferably of 1 to 10 mL/h and most preferably of 5 mL/h.

As will be clear to the person skilled in the art, the amount of time that it takes for each droplet to flow through the delay line depends on the length of the delay line, its inner diameter and the flow rate through the delay line. In one embodiment of the invention, the length and the inner diameter of the off-chip delay line (4), as well as the flow rate through the delay line (4) can be adjusted to provide a flow time of the droplets (5) from the beginning to the end of the delay line (4), which can also be defined as the incubation time, of 0.1 s to 100 h, preferably of 1 s to 48 h, more preferably of 10 s to 24 h, even more preferably of 1 min to 12 h, yet more preferably of 2 min to 6 h, yet more preferably of 5 min to 5 h, yet more preferably of 10 min to 4 h, yet more preferably of 15 min to 3 h, yet more preferably of 30 min to 2 h, and most preferably of 1 h.

For certain types of assays to be performed inside the droplets, such as cell growth or nucleic acid amplification, it may be necessary to control the incubation conditions of the droplets while they are flowing through the delay line. In one embodiment therefore, the microfluidic system of the invention is configured to allow to control the incubation conditions of at least part of the delay line (4). Preferably, the system is configured to allow to control the incubation conditions of the entire or essentially the entire delay line (4). In one embodiment, the system is configured to provide one uniform incubation condition along at least 20 % of the length of the delay line (4), more preferably along at least 40 %, even more preferably along at least 60 %, yet more preferably along at least 80 % and most preferably essentially along the entire delay line (4). In an alternative embodiment, the system is configured to provide at least two, preferably at least three, more preferably at least four and most preferably at least five different incubation conditions along the length of the delay line (4).

In one embodiment of the invention, the incubation conditions which the microfluidic device is configured to allow to control along at least part of the delay line (4) are selected from the group comprising the temperature, the concentration of any molecule that is able to diffuse through the wall of the off-chip delay line (4), the magnetic field, the electric field and any combination thereof. In a preferred embodiment, the system is configured to permit the variation of at least two incubation conditions independently from each other along the length of the delay line (4).

In one embodiment, the system is configured to allow to maintain a uniform temperature along at least part of the delay line (4). Preferably, the system is configured to maintain a uniform temperature along the delay line (4) of between 0°C to 110°C, preferably between 4°C to 90°C, more preferably between 8°C to 80°C, even more preferably between 12 to 70°C, yet more preferably between 16 to 60°C, even more preferably between 20°C to 50°C, even more preferably between 25°C to 45°C, even more preferably between 30°C to 40°C and most preferably 37°C.

In a preferred embodiment, the system comprises a means for controlling the incubation temperature in at least part of the delay line (4), preferably this means is selected from the group comprising a bed of heated metal beads, a water bath and a peltier element. The person skilled in the art is able to determine how best to maintain the required temperature of the part of the delay line with one of these elements.

In one embodiment, the microfluidic system is configured to allow to control the incubation conditions along the delay line (4) in such a way that the droplets (5) undergo thermal cycling as they flow through the delay line (4). In a preferred embodiment, the thermal cycling that the droplets (5) undergo can be adjusted to allow nucleic acid amplification by an amplification reaction such as isothermal amplification, PCR or real time PCR.

For some applications, it may be necessary to maintain or vary the concentration of certain constituents, such as gases, in the droplets (5) during the incubation. This can be achieved by using a delay line wall through which such constituents can diffuse and by surrounding the delay line with the required concentration of the constituent(s) in question. In a preferred embodiment therefore, the microfluidic system comprises means that allow to control the concentration around at least part of the delay line (4) of molecules that are able to diffuse through the wall of the delay line (4). Depending on the material of the delay line wall, such molecules may be gases. Preferably, the gases are CO₂, O₂, and N₂. The person skilled in the art will be able to select the material with the desired property to allow such diffusion.

In one embodiment of the invention, device a) of the microfluidic system is configured to generate or provide droplets (5) that each comprise at least one solvent and at least one additional component such as biological material. More preferably, it is configured to generate or provide droplets (5) that comprise at least one solvent, at least one biological material such as a living cell, and at least one substrate with a property that can be detected by the device c) of the microfluidic system.

In a preferred embodiment, the solvent is water.

In one embodiment of the microfluidic system, the device a) is configured to generate droplets (5) to be directly injected into the delay line (4). In an alternative embodiment, the system comprises a droplet nanoinjection device (2), which is configured to inject an additional component to a preformed droplet. In a further alternative embodiment, the device a) is a droplet reinjection device which is configured to inject droplets (5) that were previously formed into the system. In a further alternative embodiment, the device a) is a droplet fusion device which is configured to fuse two pre-existing droplets together to form a larger droplet that comprises the constituents of both of the fused droplets. In further alternatives, the microfluidic device comprises more than one of these devices. It will be clear to the person skilled in the art which are compatible.

In one embodiment of the invention, the device a) of the microfluidic system is a device that is configured to use acoustic wave technology to add one or several reagents to droplets (5).

In one embodiment, the device a) of the microfluidic device is configured to generate a population of droplets (5) which differ from each other by the concentration of at least one of the components of the droplets (5). In a preferred embodiment, device a) is configured to generate individual droplets (5) or groups of droplets (5) that all have the same concentration of one or several components but wherein the individual droplets (5) or groups of droplets (5) differ from each other in the concentration or nature of another component.

In one embodiment, the droplet-analysis device c) (7) of the microfluidic system is configured to image individual droplets (5) and/or to measure a property of individual droplets (5). Preferably the droplet-analysis device c) (7) is configured to measure a property of individual droplets (5) selected from the group comprising fluorescence, light absorption and light scattering.

One embodiment relates to the use of any of the microfluidic systems of the invention for high throughput screening or *in vitro* evolution. A further embodiment relates to the use of a microfluidic system of the invention for cell encapsulation and analysis, drug discovery, protein crystallisation, nanoparticle preparation, PCR amplification, synthesis biology, biological reactions, chemical reactions, and cell-based assays.

The microfluidic system of the invention can be used to perform high throughput screening methods or *in vitro* evolution methods in which the incubation time and conditions can be precisely controlled and are uniform across the different droplets. This allows to decrease the variability in the experimental setup and to thereby provide more reliable methods that are subject to less variation than in the prior art.

In a further embodiment therefore, the invention is a method for high throughput screening or *in vitro* evolution which comprises the steps of:
i. providing a microfluidic system of the invention,
ii. generating or providing droplets (5) by using the device a) of the microfluidic system,
iii. incubating the droplets (5) generated or provided in step ii. by passing them through the off-chip delay line b) (4) of the microfluidic system,
iv. after the incubation of step iii., measuring a property of, or imaging, individual droplets (5) with the droplet-analysis device c) (7) of the microfluidic system,
v. optionally isolating a droplet (5) or a population of droplets (5) that have a desired property with the device d) of the microfluidic system,
wherein the incubation time of each of the droplets (5) from the moment of entry into the delay line (4) until the moment of exit of the delay line (4) is essentially the same, wherein the droplets (5) generated in step ii. comprise a material to be analysed, preferably the material to be analysed is a natural polymer selected from the group comprising DNA, RNA, peptides, proteins, or a combination thereof.

As will be clear to the person skilled in the art, since the device a) of the microfluidic system generates or provides droplets (5) that either have an unconstrained diameter that is the same or larger than the inner diameter of the off-chip delay line (4), or that are separated by droplets with such a diameter, the droplets flow within the off-chip delay line (4) in a single file, in the order in which they are introduced into the delay line (4). This ensures that all the droplets (5) are incubated essentially for the same amount of time while. It also allows keeping track of the droplets (5) as they are not mixed during incubation.

In one embodiment, the separating-droplets (6), or plugs, that are generated or inserted between droplets (5) or groups of droplets (5) with a diameter that is smaller than the inner diameter of the delay line (4), are made of any phase that is immiscible with the phase of the droplets (5). If the droplets (5) are made of an aqueous phase for example, the separating-droplets (6) can be essentially made of an oil phase or a gas phase. An oil phase can for example essentially comprise a mineral oil, a fluorocarbon oil, a silicon oil, a hydrocarbon oil, a vegetable oil or any combination thereof. In a preferred embodiment, the separating droplets (6) are made of a mineral oil.

In the context of this invention, an incubation time that is essentially the same for all the droplets (5) is an incubation time that varies across the population of droplets (5) by 20 % or less, more preferably by 15 % or less, even more preferably by 10 % or less, yet more preferably by 5 % or less, yet more preferably by 3 % or less and most preferably by 1 % or less.

The droplets (5) generated or provided in step ii. of the method according to the invention are either
1) droplets (5) with a diameter that is equal or larger than the inner diameter of the off-chip delay line (4); or
2) droplets (5) with a diameter that is smaller than the inner diameter of the off-chip delay line (4), in which case the device a) in addition generates and/or inserts, between each droplet (5) or group of about 2 to about 100 droplets (5), a separating-droplet (6) with a diameter that is equal or larger than the inner diameter of the off-chip delay line (4).

This ensures that the droplets (5) flow inside the delay line (4) in a single file. As a result, the incubation time of each of the droplets (5) from the moment of entry into the delay line (4) until the moment of exit of the delay line (4) is essentially the same.

In a preferred embodiment of the method according to the invention, when the diameter of the droplets (5) is smaller than the inner diameter of the off-chip delay line (4), a separating-droplet (6) with a diameter that is equal or larger than the inner diameter of the off-chip delay line (4) is generated or inserted between each droplet (5) prior to the incubation of step iii.

In one embodiment of the method of the invention, the droplets (5) generated or provided in step ii. comprise a material to be analysed, preferably the material to be analysed is a biological material.

In a preferred embodiment of the invention, the biological material is:
1) a natural polymer selected from the group comprising nucleic acids, peptides, proteins, or a combination thereof; or
2) a cell or a group of cells selected from the group comprising eukaryotic cells, bacteria, fungi, algae, actinomycetes, or a combination thereof.

Preferred nucleic acids are DNA and RNA.

Preferably, the eukaryotic cells comprised in the droplets (5) are mammalian cells, insect cells, or yeast cells. Preferred mammalian cells are human cells and mouse cells.

Preferably, the fungi that are comprised in the droplets (5) are filamentous fungi.

In one embodiment of the invention, at least some of the droplets (5) generated or provided in step ii. comprises two types of cells.

When at least some of the droplets (5) generated or provided in step ii. comprise DNA, they preferably also comprise reagents for amplification of the DNA. It will be clear to the person skilled in the art which types of reagents allow such amplification. In one example, the droplets (5), in addition to DNA, also comprise a polymerase, dNTPs, a forward primer a reverse primer and optionally a probe, which can be a fluorescent probe such as a TaqMan probe. When the droplets (5) generated or provided in step ii. comprise DNA and regents for amplification of the DNA, the device is preferably configured to provide the necessary incubation conditions for DNA amplification as the droplets (5) flow through the off-chip delay line (4). The type of incubation condition for which the delay line is configured depends on the type of amplification that is performed. It will for example be clear to the person skilled in the art that for performing PCR, the device has to be configured to provide temperature cycles as the droplets flow through the delay line in order for the PCR reaction to take place inside the droplets. In contrast, if the amplification is an isothermal amplification, the droplets should be exposed to a constant temperature during incubation.

In one embodiment of the invention, at least a subset of the droplets (5) generated in step ii. comprise a substrate with a property that is measurable by the droplet-analysis device c) (7). Such a substrate is preferably selected from the group comprising a fluorescent assay substrate, a pH assay substrate, a light absorption assay substrate, a mass spectrum assay substrate, an image-based assay substrate, or a precursor of any such substrates. Preferably the property of the substrate that is measurable by device c) varies in response to a particular condition inside the droplet. As way of example, the substrate can be a molecule that changes colour in function of the pH of the droplet.

In one embodiment of the invention, at least a subset of the droplets (5) generated in step ii. comprise a living organism that is able to produce a molecule with a property that is measurable by the droplet-analysis device c) (7). The living organism is preferably a bacteria or a yeast that is able to produce a fluorescent protein such as GFP, YFP, RFP, CFP. In such as case, the device c) (7) is preferably configured to detect fluorescence of the protein produced by the living organism. The living organism is preferably auxotrophic.

In one embodiment of the invention, at least one of the components of at least a subset of the droplets (5) generated in step ii. varies in concentration or property across at least a subset of the droplets (5). In one embodiment, different droplets (5) or populations of droplets (5) generated or provided in step ii. differ from each other by the concentration of one component. In another embodiment, different droplets (5) or populations of droplets (5) all comprise a different reagent. Such an embodiment is for example useful to screen a library of reagents for their effect on a particular type of biological cell or cell system. In another embodiment, different droplets (5) or populations of droplets (5) generated or provided in step ii. differ in that at least some of them comprise cells that carry one or several randomly introduced mutations. This embodiment for example allows screening a large number of individual clones to find a mutation that provides its host with a property of interest. The different embodiments described in this paragraph can also be combined as required. Different droplets (5) or populations of droplets (5) can for example vary both in the concentration of a reagent and the nature of a biological material as required for the assay.

In a preferred embodiment, at least a subset of the droplets (5) generated or provided in step ii. comprise a cell and a substrate with a property that can be measured by the device c).

In one embodiment, the droplets (5) are separated in the delay line (4) by spacing oil, wherein the spacing oil is preferably perfluorocarbon oil.

Different incubation times in the delay line can be necessary, depending on the reagents or the processes that are being analysed. The person skilled in the art will know which incubation time is most suitable for a given application. It will be clear to the person skilled in the art that the amount of time that it takes for each droplet to flow through the delay line depends on the length of the delay line, its inner diameter and the flow rate through the delay line. The person skilled in the art will be able to adjust the different parameters of the microfluidic system in order to achieve the desired incubation time of the droplets inside of the delay line (i.e. the time it takes the droplets to flow through the delay line). In one embodiment, the droplets (5) are incubated in the off-chip delay line (4) for 0.1 s to 100 h, preferably for 1 s to 48 h, more preferably for 10 s to 24 h, even more preferably for 1 min to 12 h, yet more preferably for 2 min to 6 h, yet more preferably for 5 min to 5 h, yet more preferably for 10 min to 4 h, yet more preferably for 15 min to 3 h, yet more preferably for 30 min to 2 h, and most preferably for 1 h.

For certain applications, such as cell growth or nucleic acid amplification, it may be necessary to control the incubation conditions of the droplets while they are flowing through the delay line. In one embodiment of the method of the invention therefore the delay line (4) is maintained at a uniform temperature or different parts of the delay line (4) are maintained at different temperatures along at least part of the delay line (4). The person skilled in the art will be able to determine which temperature or temperatures are most suitable, depending on the application. If the incubation inside the delay line is meant to support cell growth, the person skilled in the art would know to maintain at least part of the delay line at the required temperatures. For human cells or E. coli cells for example, the optimal temperature would likely be 37 °C. In contrast, if the method involves a PCR amplification of DNA inside the droplets, the method may require the droplets to undergo thermal cycling as they flow through the delay line. Such a step can also be part of the method of this invention.

For some applications, it is necessary to maintain or vary the concentration of certain constituents, such as gases, in the droplets during the incubation in the delay line. This can be achieved by using a delay line wall through which such constituents can diffuse and by surrounding the delay line with the required concentration of the constituent(s) in question. In a preferred embodiment therefore, the method of the invention comprises controlling the concentration inside the droplets (5) of molecules that are able to diffuse through the wall of the delay line (4). Depending on the material of the delay line wall, such molecules may be gases. Preferably, the gases are CO₂, O₂, and N₂. The person skilled in the art will be able to select the material with the desired property to allow such diffusion.

In one embodiment of the method of the invention, the droplets (5) that are generated or provided in step ii comprise at least one solvent and at least one additional component, such as a biological material. More preferably, the droplets (5) generated or provided in step ii. of the method comprise at least one solvent (such as water), at least one biological material such as a living cell, and at least one substrate with a property that can be detected by the device c) of the microfluidic.

In one embodiment of the method of the invention, the droplets (5) generated in step ii. are directly injected into the delay line (4) without any further addition of reagents. In an alternative embodiment, at least a subset of droplets (5) are first generated or provided and are then nanoinjected with a further component before being injected into the delay line (4). In a further alternative embodiment, droplets (5) that were previously generated, and optionally incubated, are injected into the delay line (4) by a reinjection device. In a further alternative embodiment, step ii. comprises fusing two pre-existing droplets together to form a larger droplet that comprises the constituents of both of the fused droplets. In further alternatives, step ii. of the method comprises two or more of the steps described in this paragraph.

In one embodiment of the invention, step ii. results in a population of droplets (5) which differ from each other by the concentration of at least one of the components of the droplets (5). In a preferred embodiment, the individual droplets (5) generated or provided in step ii. all have the same concentration of one or several components but individual droplets (5) or groups of droplets (5) differ from each other in the concentration or nature of another component.

In one embodiment of the invention, step iv. of the method consists in imaging the individual droplets (5). In an alternative embodiment, step iv. consists in measure a property of the individual droplets (5). Preferably the property of the individual droplets (5) that is measured is selected from the group comprising fluorescence, light absorption and light scattering.

In one embodiment of the invention, the method comprises a step of isolating a droplet (5) or a population of droplets (5) that have a desired property as measured by the device c) of the microfluidic device of the invention.

### FIGURE CAPTIONS

**Figure 1****.** Drawing of typical design of mask for a droplet generating device (1).
**Figure 2****.** Drawing of a typical design for a droplet nanoinjection device (2).
**Figure 3****.** Picture of the process of nanoinjection.
**Figure 4****.** Picture of droplets (5) moving in a delay line tubing (4) in a single file during incubation. The diameter of the droplets (5) is larger than the inner diameter of the delay line tubing (4).
**Figure 5****.** Picture of droplets (5) moving in a delay line tubing (4) in a single file during incubation. The diameter of the assay droplets (5) is smaller than the inner diameter of the delay line tubing (4), but these droplets (5) are separated by separating-droplets (plugs) (6).
**Figure 6****.** Drawing of a typical design for a droplet analysis device (7) after incubation.
**Figure 7****.** Drawing of an optical setup for droplet analysis.

The following non-limiting examples are provided to illustrate the invention.

### EXAMPLES

### Example 1: Preparation of a droplet generating device (1)

Soft-lithography in poly(dimenthylsiloxane) (PDMS) was used to prepare the droplet generating device (1). A SU-8 photoresist mould was used to prepare the PDMS. To prepare the SU-8 mold, a layer of SU-8 was spin coated on a silicon wafer. The wafer was covered by a designed mask and exposed to UV for a certain period of time. After full development and baking the wafer, the SU-8 mould was ready for PDMS. The SU-8 thickness for droplet making chip in this example was 200 µm. The droplet volume generated by the chip depends on the SU-8 thickness. To generate nanoliter droplets, the thickness can vary from 80 µm to 500 µm.

The thickness of the SU-8 mould for different types of PDMS chip is varies. The SU-8 thickness for droplet nanoinjection chip, droplet sorting chip can for example respectively be 180 µm and 350 µm.

The droplet volume generated by the chip depends on the SU-8 thickness. To generate nanoliter droplets, the thickness can vary from 80 µm to 500 µm.

After preparation of the SU-8 mould, PDMS was casted on the mould and bound to a glass side. The inside part of microfluidic channel was treated by a commercial surface coating agent (Trichloro-(1H,1H,2H,2H-perfluorooctyl)-silane, Sigma-Aldrich) to make the channel surface hydrophobic.

### Example 2: Generation of fungi spore-containing droplets

To generate droplets on a chip, the PDMS chip was connected via tubing to an oil phase reservoir, an aqueous phase reservoir and an outlet tubing. A possible chip design is shown in figure 1. In this case, the oil phase consists of perfluorocarbon oil (HFE7500, 3M) with 5% (w/w) of a surfactant, made by coupling oligomeric perfluorinated polyethers (PFPE) with polyethyleneglycol (PEG) (Biocompatible surfactants for water-in-fluorocarbon emulsions, Lab Chip, 2008, 8, 1632-1639). However, any phase that is immiscible with the droplets, which in this case are made of an aqueous phase, could have been used (any oil or gas phase). The aqueous phase consists of fungi spore suspension as an example, but is not limited to fungi spores. In other examples, mammalian cells, bacterial cells, yeast cells etc. could be used. The flow rate was controlled by syringe pumps (PHD2000, Havard Apparatus). The flow rate of oil phase was 4 mL/h, and the flow rate of aqueous phase was 3 mL/h. The droplet volume generated here was 20 nL (diameter = 0.336 mm). The droplets encapsulate the fungi spores during the droplet generation process. The droplets were collected in a vial and incubated at 30 °C over 48 hours for germination and growth of the fungi.

### Example 3: Nanoinjection of a fluorescent assay substrate into the droplets

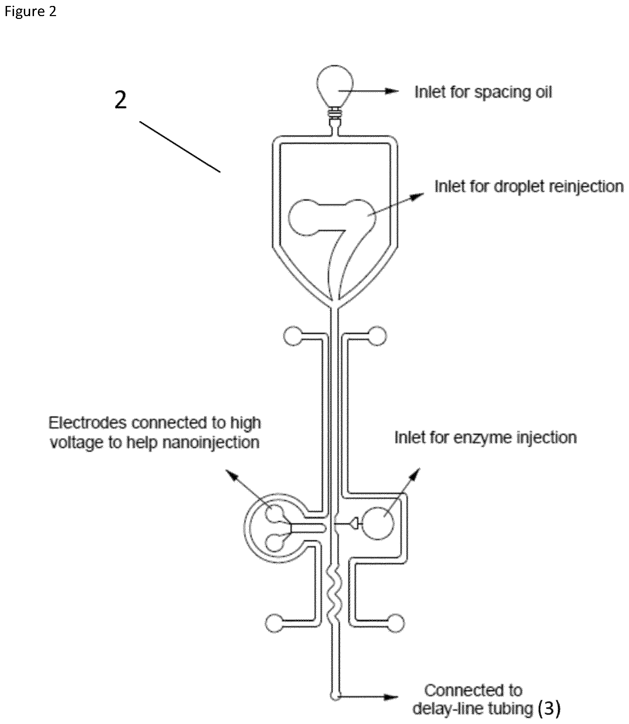

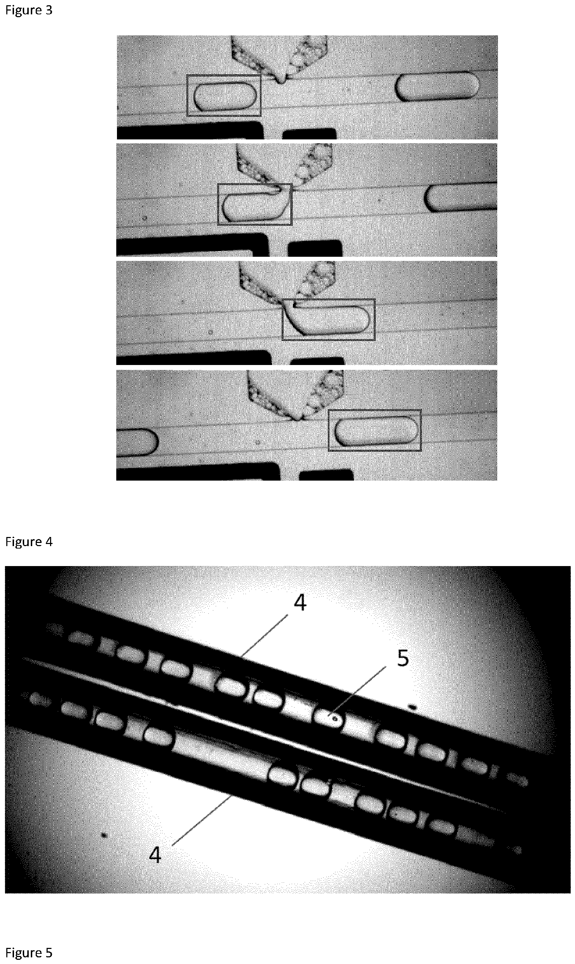

After incubation, the droplets were reinjected into a chip that is capable of nanoinjection. Nanoinjection is employed in order to add the fluorescent assay substrate directly prior to the start of the assay reaction into droplets. A typical design of nanoinjection device (2) is shown in figure 2 and a typical nanoinjection process is pictured in figure 3. The flow rate of spacing oil was 0.6 mL/h, the flow rate of droplet reinjection was 0.5 mL/h, and the flow rate of aqueous phase for nanoinjection was 0.1 mL/h. A high voltage with 20,000 V and 20,000 Hz was added to help nanoinjection. Other technologies, such as acoustic wave technology can also be used to add reagents to droplets. The spacing oil phase consists of perfluorocarbon oil. The droplets contain the grown fungi after 48 hours of incubation. The aqueous phase for nanoinjection contains a fluorescent enzyme substrate.

A poly(tetrafluoroethylene) (PTFE) tubing (inner diameter = 0.3 mm, outer diameter = 0.56 mm) was connected to the outlet (3) of the nanoinjection device (2). The tubing is a delay line (4) in which the droplets that comprise the fungi and the fluorescent enzyme substrate are all incubated for a precisely controlled time by moving through the delay line (4) in a single file.

### Example 4: Temperature control of droplet incubation

After nanoinjection, the droplets flowed in the order in which they were injected into the PTFE tubing (the delay line (4)). The droplets were continuously moving in the tubing. The length of the tubing in this example was 6 meters, but can also be significantly shorter or longer (e.g. up to 100 m) depending on the incubation time needed. The tubing was incubated at 30 °C in the present example. The temperature setting however, can be adapted to the needs of each specific assay. Temperature control was obtained by submerging the tubing containing the droplets for assay incubation into a bed of heated metal beads or in a water bath. Other arrangements like a tubing coil surrounding a peltier element could be another option.

### Example 5: Droplet incubation inside the delay line tubing (4)

There are two scenarios for the droplet incubation in the delay line tubing (4). In the present case, the diameter of the droplets is larger than the inner diameter of the PTFE tubing, the droplets were therefore flowing inside the tubing one-by-one in a linear sequential order that allows exact control of start and end of the reaction, i.e. timing of the assay incubation period (as shown in figure 4). Therefore, the diversity of incubation time for each droplet is low for nanoliter droplets when using this method. This is much more accurate than the simple collection of droplets following injection of the assay substrate and the pooled incubation of droplets e.g. in an Eppendorf vial without a defined synchronized start and stop of the assay in each droplet.

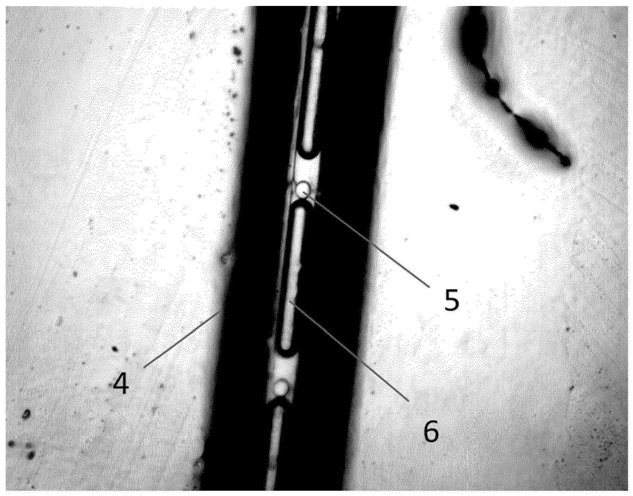

In the case when the droplet diameter is smaller than the inner diameter of tubing, the droplets would not in normal circumstances flow inside the tubing one-by-one in a linear sequential order. Due to the phenomenon of laminal flow in the tubing, the droplets at the centre of tubing move faster than the droplets at the side of the tubing. Therefore, the incubation time of the different droplets in the tubing cannot be well controlled by the "tubing incubation method". To keep the smaller droplets in a linear sequential order, we introduced a third phase plug (separating-droplet (6)) to space out the smaller droplets (as shown in figure 5). The third phase plug can be any phase which is immiscible and stable with the droplets and spacing oil phase. Possible choices of the third phase are for example a mineral oil phase or an air phase. The diameter of the third phase plug is larger than the inner diameter of the tubing. In this case, we chose to use separating plugs of 20 nL, but in theory they could have been of a volume of about 10 nL to about 10000 nL with the kind of tubing used for this experiment. The third phase plug was introduced into the tubing when the smaller droplets are entering the tubing. The small droplets were well spaced by the plugs and between each plug there can be 1-100 small droplets.

### Example 6: Droplet analysis after incubation

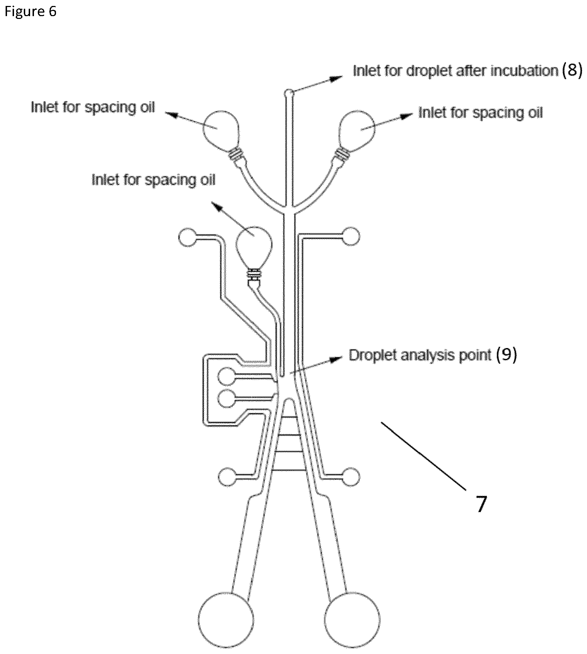

Following incubation in the delay line tubing, the droplets were injected into a droplet analysis device/chip (7) (figure 6). After incubation in the tubing, the droplets were continuously reinjected to the droplet analysis device (7). In the present case, the spacing oil consists of perfluorocarbon oil and the flow rate of the spacing oil was 1-4 mL/h, but this rate has to be adjusted depending on the experiment. The droplets were moved to the droplet analysis point (9) for the detection of fluorescence intensity following the enzyme reaction.

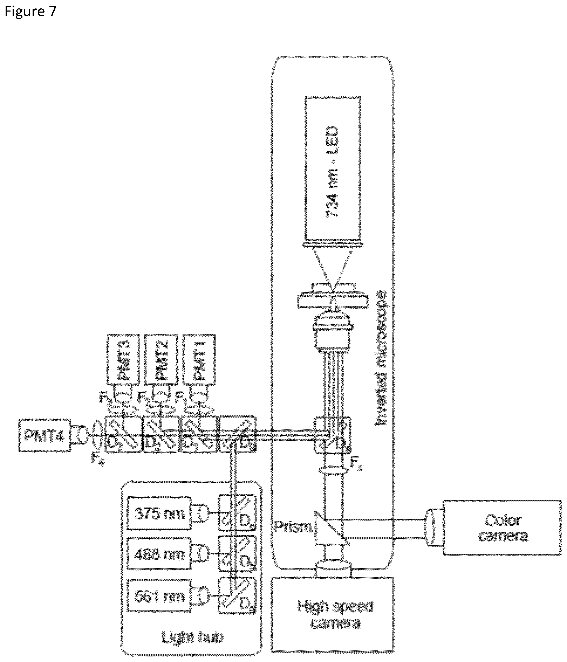

The detection was performed by an optical setup as shown in figure 7. The optical setup consisted of a TI-U inverted microscope (Nikon) which was mounted on an optical platform (TMC). A highspeed camera (Mikrotron) and a color camera (Nikon) were mounted on the inverted microscope to record droplet movement in the microfluidic chip. Three lasers (a 20mW/375 nm Luxx diode laser, a 80 mW/488 nm Luxx diode laser and a 150 mW/561 nm Coblot Jive DPSS laser, Omicron) were used to excite the fluorophores contained in the droplets. The three lasers were reflected by a dichroic beamsplitter into the microscope. Inside the microscope, the lasers were reflected at a beamsplitter and focused into the microfluidic channel by a 20x objective. The fluorescence emitted from droplets was reflected by the beamsplitter, filtered by appropriate set of optical filters (Semrock) and then collected with photomultiplier tubes (Hammamatsu). The signal from photomultiplier tubes was recorded by a data-acquisition system (National Instruments).

## Claims

1. A microfluidic system comprising:
a) a chip comprising a droplet-generating device (1) and/or a droplet nanoinjection device (2) and/or a droplet reinjection device and/or a droplet fusion device,
b) an off-chip delay line (4),
c) a droplet-analysis device (7), and
d) optionally a droplet sorting device,
wherein the off-chip delay line (4) is fluidically connected at one end to the outlet (3) of the device a), and at the other end to the inlet (8) of the device c), **characterized in that** the device a) is configured to generate or provide either:
1) droplets (5) with an unconstrained diameter that is equal or larger than the inner diameter of the off-chip delay line (4); or
2) droplets (5) with an unconstrained diameter that is smaller than the inner diameter of the off-chip delay line (4), in which case the device a) is configured in addition to generate and/or insert, between each droplet (5) or group of about 2 to about 100 droplets (5), a separating-droplet (6) with an unconstrained diameter that is equal or larger than the inner diameter of the off-chip delay line (4),
wherein the inner diameter of the off-chip delay line (4) is constant over its entire length, and
wherein the inner diameter of the off-chip delay line (4) is between 0.01 mm to 10 mm, preferably between 0.05 mm to 5 mm.

2. The microfluidic system according to claim 1, wherein the length of the off-chip delay line (4) is between 0.1 m to 100 m, preferably between 0.5 m to 80 m.

3. The microfluidic system according any of the preceding claims, wherein the material of the off-chip delay line (4) is selected from the group comprising glass, PTFE (polytetrafluoroethylene), PEEK (polyetheretherketone), FEP (fluorinated ethylene-propylene), ETFE (ethylene tetrafluoroethylene), PP (polypropylene) and any combination thereof.

4. The microfluidic system according any of the preceding claims, wherein the system is configured to provide a flow rate through the off-chip delay line (4) of 0.001 mL/h to 100 mL/h, preferably of 0.05 mL/h to 50 mL/h.

5. The microfluidic system according any of the preceding claims, wherein the length of the off-chip delay line (4), the inner diameter of the delay line (4) and the flow rate through the delay line (4) can be adjusted to provide a transit time of the droplets (5) through the delay line (4) of 0.1 s to 100 h, preferably from 1 s to 48 h.

6. The microfluidic system according any of the preceding claims, wherein the system is configured to control the incubation conditions of at least part of the delay line (4).

7. The microfluidic system according to claim 6, wherein the system comprises a means for controlling the incubation temperature in at least part of the delay line (4), wherein this means is preferably selected from the group comprising a bed of heated metal beads, a water bath and a peltier element.

8. The microfluidic system of any of the preceding claims, wherein the device a) is configured to generate droplets (5) that each comprise at least one solvent and at least one additional component such as a biological material.

9. The microfluidic system of any of the preceding claims, wherein the droplet-analysis device c) (7) is configured to image individual droplets (5) and/or to measure a property of individual droplets (5) selected from the group comprising fluorescence, light absorption and light scattering.

10. Method for high throughput screening or *in vitro* evolution comprising the steps of:
i. providing a microfluidic system of any of claims 1 to 9,
ii. generating or providing droplets (5) by using the device a) of the microfluidic system,
iii. incubating the droplets (5) generated or provided in step ii. by passing them through the off-chip delay line b) (4) of the microfluidic system,
iv. after the incubation of step iii., measuring a property of, or imaging, individual droplets (5) with the droplet-analysis device c) (7) of the microfluidic system,
v. optionally isolating a droplet (5) or a population of droplets (5) that have a desired property with the device d) of the microfluidic system,
wherein the incubation time of each of the droplets (5) from the moment of entry into the delay line (4) until the moment of exit of the delay line (4) is essentially the same,
wherein the droplets (5) generated in step ii. comprise a material to be analysed, preferably the material to be analysed is a natural polymer selected from the group comprising DNA, RNA, peptides, proteins, or a combination thereof.

11. The method of claim 10, wherein the droplets (5) generated in step ii. comprise either
1) a substrate with a property that is measurable by the droplet-analysis device c) (7) wherein the substrate is preferably selected from the group comprising a fluorescent assay substrate, a pH assay substrate, a light absorption assay substrate, a mass spectrum assay substrate, an image-based assay substrate, a precursor of any such substrates and a combination of any such substrates; and/or
2) a living organism that is able to produce a molecule with a property that is measurable by the droplet-analysis device c) (7), wherein the living organism is preferably a bacteria or a yeast that is able to produce a fluorescent protein such as GFP, YFP, RFP, CFP.

12. The method of any of claims 10 and 11, wherein at least one of the components of the droplets (5) generated in step ii. varies in concentration or property across individual droplets (5) or populations of droplets (5).

## Patentansprüche

1. Mikrofluidisches System, umfassend:
a) einen Chip mit einer Tropfen-Erzeugungsvorrichtung (1) und/oder einer Tropfen-Nanoinjektionsvorrichtung (2) und/oder einer Tropfen-Reinjektionsvorrichtung und/oder einer Tropfen-Fusionsvorrichtung,
b) eine chipexterne Verzögerungsleitung (4),
c) eine Tropfen-Analysevorrichtung (7), und
d) optional eine Tropfen-Sortiereinrichtung,
wobei die chipexterne Verzögerungsleitung (4) an einem Ende mit dem Auslass (3) der Vorrichtung a) und am anderen Ende mit dem Einlass (8) der Vorrichtung c) fluidisch verbunden ist, **dadurch gekennzeichnet, dass** die Vorrichtung a) so konfiguriert ist, dass entweder:
1) Tropfen (5) mit einem uneingeschränkten Durchmesser, der gleichgroß oder größer als der Innendurchmesser der chipexternen Verzögerungsleitung (4) ist; oder
2) Tropfen (5) mit einem uneingeschränkten Durchmesser, der kleiner ist als der Innendurchmesser der chipexternen Verzögerungsleitung (4), wobei in diesem Fall die Vorrichtung a) zusätzlich konfiguriert ist, zwischen jedem Tropfen (5) oder Gruppe von etwa 2 bis etwa 100 Tropfen (5), ein Trenntropfen (6) mit einem uneingeschränkten Durchmesser, der gleichgroß oder größer als der Innendurchmesser der chipexternen Verzögerungsleitung (4) ist, zu erzeugen und/oder einzufügen,
wobei der Innendurchmesser der chipexternen Verzögerungsleitung (4) über ihre gesamte Länge konstant ist, und
wobei der Innendurchmesser der chipexternen Verzögerungsleitung (4) zwischen 0,01 mm bis 10 mm, vorzugsweise zwischen 0,05 mm bis 5 mm beträgt.

2. Mikrofluidisches System nach Anspruch 1, **dadurch gekennzeichnet, dass** die Länge der chipexternen Verzögerungsleitung (4) zwischen 0,1 m bis 100 m, vorzugsweise zwischen 0,5 m bis 80 m beträgt.

3. Mikrofluidisches System nach einem der vorhergehenden Ansprüche, wobei das Material der chipexternen Verzögerungsleitung (4) ausgewählt ist aus der Gruppe umfassend Glas, PTFE (Polytetrafluorethylen), PEEK (Polyetheretherketon), FEP (fluoriertes Ethylen-Propylen), ETFE (Ethylen-Tetrafluorethylen), PP (Polypropylen) und jede Kombination davon.

4. Mikrofluidisches System nach einem der vorhergehenden Ansprüche, wobei das System konfiguriert ist eine Flussrate durch die chipexterne Verzögerungsleitung (4) von 0,001 ml/h bis 100 ml/h, vorzugsweise von 0,05 ml/h bis 50 ml/h, bereitzustellen.

5. Mikrofluidisches System nach einem der vorhergehenden Ansprüche, wobei die Länge der chipexternen Verzögerungsleitung (4), der Innendurchmesser der Verzögerungsleitung (4) und die Flussrate durch die Verzögerungsleitung (4) eingestellt werden können, um eine Durchflusszeit der Tropfen (5) durch die Verzögerungsleitung (4) von 0,1 s bis 100 h, vorzugsweise von 1 s bis 48 h, bereitzustellen.

6. Mikrofluidisches System nach einem der vorhergehenden Ansprüche, wobei das System konfiguriert ist, um die Inkubationsbedingungen von mindestens einem Teil der Verzögerungsleitung zu steuern (4).

7. Mikrofluidisches System nach Anspruch 6, wobei das System ein Mittel zum Steuern der Inkubationstemperatur in mindestens einem Teil der Verzögerungsleitung (4) umfasst, wobei dieses Mittel vorzugsweise ausgewählt ist aus der Gruppe umfassend ein Bett aus erhitzten Metallkügelchen, ein Wasserbad und ein Peltier-Element.

8. Mikrofluidisches System nach einem der vorhergehenden Ansprüche, wobei die Vorrichtung a) konfiguriert ist, um Tropfen (5) zu erzeugen, die jeweils mindestens ein Lösungsmittel und mindestens eine zusätzliche Komponente, wie ein biologisches Material, umfassen.

9. Mikrofluidisches System nach einem der vorhergehenden Ansprüche, wobei die Tropfen-Analysevorrichtung c) (7) konfiguriert ist, um einzelne Tropfen (5) abzubilden und/oder eine Eigenschaft einzelner Tropfen (5), ausgewählt aus der Gruppe umfassend Fluoreszenz, Lichtabsorption und Lichtstreuung, zu messen.

10. Verfahren zum Hochdurchsatz-Screening oder in vitro-Evolution, umfassend die Schritte:
i. Bereitstellen eines mikrofluidischen Systems nach einem der Ansprüche 1 bis 9,
ii. Erzeugung oder Bereitstellung von Tropfen (5) unter Verwendung der Vorrichtung a) des mikrofluidischen Systems,
iii. Inkubieren der in Schritt ii. erzeugten oder bereitgestellten Tropfen (5) indem sie durch die chipexterne Verzögerungsleitung b) (4) des mikrofluidischen Systems geführt werden,
iv. nach der Inkubation von Schritt iii., Messen einer Eigenschaft oder Abbildung einzelner Tropfen (5) mit der Tropfen-Analysevorrichtung c) (7) des mikrofluidischen Systems,
v. optionales Isolieren eines Tropfens (5) oder einer Population von Tropfen (5), die eine gewünschte Eigenschaft aufweisen, mit der Vorrichtung d) des mikrofluidischen Systems,
wobei die Inkubationszeit jedes der Tropfen (5) vom Eintrittszeitpunkt in die Verzögerungsleitung (4) bis zum Austrittszeitpunkt aus der Verzögerungsleitung (4) im Wesentlichen gleich ist,
wobei die in Schritt ii. erzeugten Tropfen (5) ein zu analysierendes Material umfassen, vorzugsweise ist das zu analysierende Material ein natürliches Polymer ausgewählt aus der Gruppe umfassend DNA, RNA, Peptide, Proteine, oder eine Kombination davon.

11. Verfahren nach Anspruch 10, wobei die in Schritt ii. erzeugten Tropfen (5) entweder umfassen
1) ein Substrat mit einer Eigenschaft, die durch die Tropfen-Analysevorrichtung c) (7) messbar ist, wobei das Substrat vorzugsweise ausgewählt ist aus der Gruppe umfassend ein Fluoreszenz-Assay-Substrat, ein pH-Assay-Substrat, ein Lichtabsorptions-Assay-Substrat, ein Massenspektrum-Assay-Substrat, ein bildbasiertes Assay-Substrat, ein Vorläufer eines solchen Substrats und eine Kombination eines beliebigen solchen Substrats; und/oder
2) ein lebender Organismus, der in der Lage ist, ein Molekül mit einer durch die Tropfen-Analysevorrichtung c) (7) messbaren Eigenschaft zu produzieren, wobei der lebende Organismus vorzugsweise ein Bakterium oder eine Hefe ist, die in der Lage ist, ein fluoreszierendes Protein wie GFP, YFP, RFP, CFP zu produzieren.

12. Verfahren nach einem der Ansprüche 10 und 11, wobei mindestens eine der Komponenten der in Schritt ii. erzeugten Tropfen (5), in Konzentration oder Eigenschaft über einzelne Tropfen (5) oder Tropfen-Populationen (5) variiert.

## Revendications

1. Un système microfluidique comprenant :
a) une puce comprenant un dispositif de génération de gouttelettes (1) et/ou un dispositif de nanoinjection de gouttelettes (2) et/ou un dispositif de réinjection de gouttelettes et/ou un dispositif de fusion de gouttelettes,
b) une ligne à retard hors puce (4),
c) un dispositif d'analyse de gouttelettes (7), et
d) éventuellement un dispositif de tri de gouttelettes, dans lequel la ligne à retard hors puce (4) est reliée fluidiquement à une extrémité à la sortie (3) du dispositif a), et à l'autre extrémité à l'entrée (8) du dispositif c), **caractérisé en ce que** le dispositif a) est configuré pour générer ou fournir soit :
1) des gouttelettes (5) avec un diamètre non contraint qui est égal ou supérieur au diamètre intérieur de la ligne à retard hors puce (4) ; ou
2) des gouttelettes (5) avec un diamètre non contraint qui est inférieur au diamètre intérieur de la ligne à retard hors puce (4), auquel cas le dispositif a) est configuré en plus pour générer et/ou insérer, entre chaque gouttelette (5) ou un groupe d'environ 2 à environ 100 gouttelettes (5), une gouttelette de séparation (6) avec un diamètre non contraint qui est égal ou supérieur au diamètre intérieur de la ligne à retard hors puce (4), dans lequel le diamètre de la ligne à retard hors puce (4) est constant sur toute sa longueur, et
dans lequel le diamètre intérieur de la ligne à retard hors puce (4) est compris entre 0,01 mm et 10 mm, de préférence entre 0,05 mm et 5 mm.

2. Système microfluidique selon la revendication 1, dans lequel la longueur de la ligne à retard hors puce (4) est comprise entre 0,1 m et 100 m, de préférence entre 0,5 m et 80 m.

3. Système microfluidique selon quelconque des revendications précédentes, dans lequel le matériau de la ligne a retard hors puce (4) est choisi dans le groupe comprenant le verre, le PTFE (polytétrafluoroéthylène), PEEK (polyéthercétone), FEP (éthylènepropylène fluoré), ETFE (éthylène tétrafluoroéthylène), PP (polypropylène) et toute combinaison de ceux-ci.

4. Système microfluidique selon l'une quelconque des revendications précédentes, dans lequel le système est configuré pour fournir un débit à travers la ligne à retard hors puce (4) de 0,001 ml/h à 100 ml/h, de préférence de 0,05 ml/h à 50 ml/h.

5. Système microfluidique selon l'une quelconque des revendications précédentes, dans lequel la longueur de la ligne à retard (4) hors puce, le diamètre intérieur de la ligne à retard (4) et le débit à travers la ligne à retard (4) peuvent être ajustés pour fournir un temps de transit des gouttelettes (5) à travers la ligne à retard (4) de 0,1 s à 100 h, de préférence de 1 s à 48 h.

6. Système microfluidique selon l'une quelconque des revendications précédentes, dans lequel le système est configuré pour contrôler les conditions d'incubation d'au moins une partie de la ligne de retard (4).

7. Système microfluidique selon la revendication 6, dans lequel le système comprend un moyen pour contrôler la température d'incubation dans au moins une partie de la ligne a retard (4), dans lequel ce moyen est de préférence choisi dans le groupe comprenant un lit de billes métalliques chauffées, un bain-marie et un élément Peltier.

8. Système microfluidique selon l'une quelconque des revendications précédentes, dans lequel le dispositif a) est configuré pour générer des gouttelettes (5) qui comprenant chacune au moins un solvent et au moins un composant supplémentaire tel qu'un matériau biologique.

9. Système microfluidique de l'une quelconque des revendications précédentes, dans lequel le dispositif d'analyse de gouttelettes c) (7) est configuré pour imager des gouttelettes individuelles (5) et/ou pour mesurer une propriété de gouttelettes individuelles (5) choisie dans le groupe comprenant la fluorescence, l'absorption de lumière et la diffusion de lumière.

10. Procédé de criblage à haut débit ou d'évolution in vitro comprenant les étapes consistant à :
i. fournir un système microfluidique de l'une quelconque des revendications 1 à 9,
ii. générer ou fournir des gouttelettes (5) en utilisant le dispositif a) du système microfluidique,
iii. incubation des gouttelettes (5) générées ou fournies à l'étape ii. en les faisant passer à travers la ligne à retard hors puce b) (4) du système microfluidique,
iv. après l'incubation de l'étape iii, mesurer une propriété des gouttelettes individuelles (5), ou en faire une image, avec le dispositif d'analyse de gouttelettes c) (7) du système microfluidique,
v. éventuellement isoler une gouttelette (5) ou une population de gouttelettes (5) qui ont une propriété souhaitée avec le dispositif d) du système microfluidique,
dans lequel le temps d'incubation de chacune des gouttelettes (5) depuis le moment de l'entrée dans la ligne à retard (4) jusqu'au moment de la sortie de la ligne à retard (4) est essentiellement le même,
dans lequel les gouttelettes (5) générées à l'étape ii. comprennent un matériau à analyser, de préférence le matériau à analyser est un polymère naturel choisi dans le groupe comprenant l'ADN, l'ARN, les peptides, les protéines, ou une combinaison de ceux-ci.

11. Procédé selon la revendication 10, dans lequel les gouttelettes (5) générées à l'étape ii. comprennent soit
1) un substrat ayant une propriété qui est mesurable par le dispositif d'analyse de gouttelettes c) (7) dans lequel le substrat est de préférence choisi dans le groupe comprenant un substrat de test fluorescent, un substrat de test de pH, un substrat de test d'absorption de lumière, un substrat de test de spectre de masse, un substrat de test basé sur l'image, un précurseur de l'un quelconque de ces substrats et une combinaison de l'un quelconque de ces substrats ; et/ou
2) un organisme vivant qui est capable de produire une molécule avec une propriété qui est mesurable par le dispositif d'analyse de gouttelettes c) (7), dans lequel l'organisme vivant est de préférence une bactérie ou une levure qui est capable de produire une protéine fluorescente telle que GFP, YFP, RFP, CFP.

12. Procédé de l'une quelconque des revendications 10 et 11, dans lequel au moins l'un des composants des gouttelettes (5) générées à l'étape ii. varie en concentration ou en propriété à travers des gouttelettes individuelles (5) ou des populations de gouttelettes (5).
